# EUROPEAN PATENT SPECIFICATION

(11) **EP 1 212 376 B1**
(45) Date of publication and mention of the grant of the patent: **01.02.2006**
(21) Application number: 00954851.2
(22) Date of filing: 08.09.2000
(51) Int. Cl.: C09B 61/00, C09B 69/10, A61K 8/00

(54) **SYNTHETIC VEGETAL MELANINS, PROCESS FOR THEIR PRODUCTION AND COMPOSITIONS CONTAINING THEM**
SYNTHETISCHE MELANINE AUF PFLANZENBASIS, VERFAHREN ZU DEREN HERSTELLUNG SOWIE DIESE ENTHALTENDE ZUSAMMENSETZUNGEN
MELANINES VEGETALES SYNTHETIQUES, PROCEDE DE PRODUCTION ASSOCIE, ET COMPOSITIONS LES CONTENANT

(30) Priority: 09.09.1999 IT MI991896
(43) Date of publication of application: 12.06.2002
(73) Proprietor: Ghisalberti, Carlo, Itaim-Bibi, CEP-04542-080 Sao Paulo, SP (BR)
(72) Inventor: Ghisalberti, Carlo, Itaim-Bibi, CEP-04542-080 Sao Paulo, SP (BR)
(74) Representative: Gislon, Gabriele
(86) International application number: PCT/IB2000/001276
(87) International publication number: WO 2001/018125

(56) References cited:
- EP-A- 0 363 792
- WO-A-92/00373
- WO-A-93/23480
- WO-A-94/12644
- WO-A-96/25920
- DE-A- 19 717 837
- FR-A- 2 704 554
- US-A- 5 122 461
- US-A- 5 384 116
- PATENT ABSTRACTS OF JAPAN vol. 015, no. 243 (C-0842), 21 June 1991 (1991-06-21) & JP 03 077813 A (RINJIRO SARUNO), 3 April 1991 (1991-04-03)
- PATENT ABSTRACTS OF JAPAN vol. 006, no. 078 (C-102), 15 May 1982 (1982-05-15) & JP 57 014517 A (SANSHO SEIYAKU KK), 25 January 1982 (1982-01-25)

## Description

### FIELD OF THE INVENTION

The present invention relates to high molecular weight synthetic vegetal melanins obtainable by polymerization of plant polyphenols.

More precisely, the present invention relates to synthetic vegetal melanins characterized by reproducible physical features, hereby obtainable by a controlled polymerization of a known amount of purified plant polyphenols, alone or in combination or in conjunction with eumelanin precursors.

The invention further relates to processes for the manufacture of said synthetic vegetal melanins either by chemical or enzymatic synthesis.

The invention further relates to the use of said synthetic vegetal melanins in cosmetics for the protection of the skin, of the mucous membranes and of hair against damages due to UV exposure and to oxidative environment, as well as their use for the manufacturing of pharmaceutical preparations and nutritional supplements exerting anti-inflammatory and immunomodulation activities.

### BACKGROUND OF THE INVENTION

Melanins are complex three-dimensional biopolymers with several biological functions including: photoreceptor shielding, thermoregulation, photoprotection, camouflage, metal chelation and free radical scavenging.

The term melanin refers to a variety of highly irregular heteropolymers containing several structural moieties, such as free carboxy, amino, hydroxy, carbonyl, methoxy, biphenolic, indolic, quinoic groups and so on.

A broad classification of these photoabsorbing biopolymers can be made on the basis of the starting monomers (the actual melanin precursors), major classes being: eumelanins, pheomelanins and allomelanins.

Eumelanins are brown-black nitrogenous pigments produced by the rearrangement and oxidation of L-dopa and its metabolites. A type of eumelanins are the "oxymelanins" exhibiting tetrahydroxy indoles units within their polymeric structure; other eumelanins are the "neuromelanin" from dopamine (which is produced L-dopa carboxylase) and its metabolite, 3,4-dihydroxycarboxylic acid, as well as by neural catecholamines and tetrahydroxyisoquinolines, e.g. occurring in the brain *substantia nigra.*

Pheomelanins are yellow-reddish pigments containing both nitrogen and sulfur atoms, which arise from the rearrangement and polymerization of cysteinyl-dopa, the condensation product of L-dopa and glutathione with further enzymatic hydrolysis. The pheomelanins generally result from the oxidative condensation of 1,4-benzothiazines; further pheomelanins are the "trichocromes" containing a bi-benzothiazinic chromophore (-S-C=C-C=N-); as well as condensed 1,4-benzothiazinalanines (e.g. in tricochrome C).

Allomelanins are highly colored pigments typically occuring in the plant kingdom, which may be further classified as "vegetal melanins" (alias "phytomelanins"), when resulting from the polymerization of plant polyphenols; and "fungi melanins" (alias "micomelanins"), such those containing naphthoic groups.

All melanins originate in nature from the common step of the conversion from monophenolic substances into the related ortho-diphenolic moiety. Noteworthy, this starting point of melanogenesis is always under strict enzymatic control.

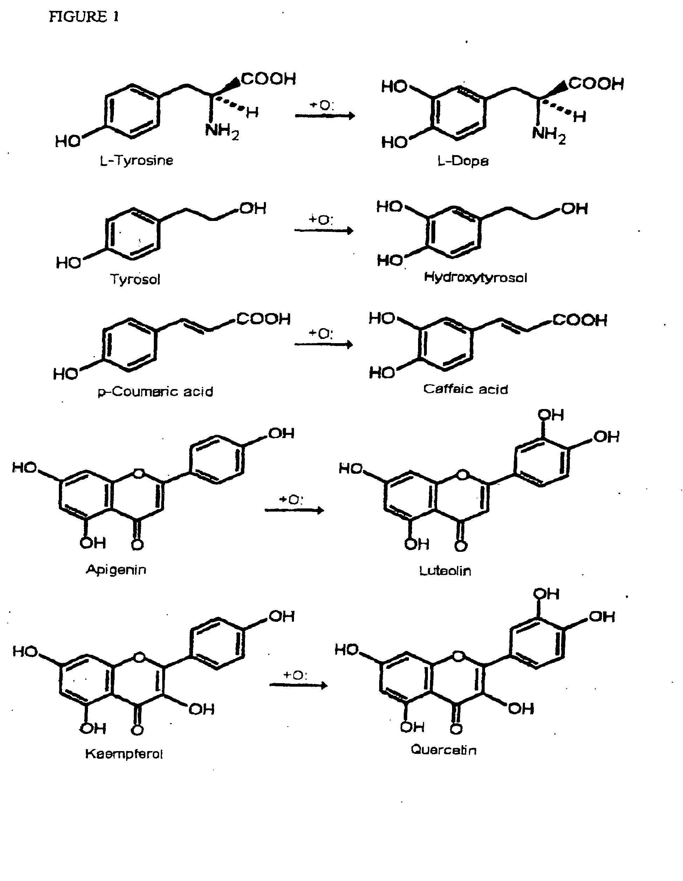

Within the plant kingdom, the hydroxylating enzymes convert the phenolic precursors in ortho-diphenols: tipically L-tyrosine to L-dopa occur in Vicia faba hull; p-hydroxybenzoic acid (PHBA) to protocatechuic acid in wheat seed hull; tyrosol to hydroxytyrosol in olive peel; p-coumaric acid to caffeic acid in green coffee skin; apigenin to luteolin in citrus peel; kaempferol to quercetin in clover blossom; pelargonin to cyanidin in grape fruit; genistein to glycitein in soybean hull, as depicted in FIG. 1 attached.

Diphenols undergo fast oxidation in presence of oxygen to provide semiquinones and quinones, which randomly polymerize and cross-link.

Some authors claim that some polymerization steps in melanogenesis may be under enzymatic control, e.g. the cyclization to indoles or decarboxylation, but the fact is quite controversial. As an example, eumelanins may be always produced by chemical synthesis, whereas the tyrosinase enzyme seems to be unable to produce eumelanin from the preformed indolic eumelanin intermediates.

The enzymatic hydroxylation of the monophenolic substrates is particularly intense during ripening or post-ripening browing of fruits and vegetables, and further enhanced under specific stress conditions such as by handling, chopping, cutting, which increase vacuoli breackages, and consequently the release of the enzymes which prompts the formation of vegetal melanins. As in the plant kingdom the polymerization occurs extracellularly, enzymes should not contribute to the final steps of the melanin polymer formation.

Melanins found increasing acceptance as ingredient in health care compositions thanks to their photoprotective, anti-ageing, radical scavenging, chelating and tanning properties.

However, vegetal melanins are not currently available for industrial applications, and industrial cosmetic and pharmaceutical applications are limited to the use of the commercially available brown-black synthetic eumelanins or sepiomelanin, a natural eumelanin which is recovered from squid or octopus inks through a complex purification process.

On the other side, there were evidences that the vegetal melanins extracted from plants may provide an excellent dermoprotection, with anti-inflammatory activity and immunomodulation, by the inhibition of the effects on paw oedema and adjuvant induced disease, as described by Avramidis N. et al. (Arzneimittelforschung, 48(7):764-71, 1998).

The synthesis of novel pseudo-melanins is described in WO96/25290. However, these are fully artificial melanins, as there are no evidences of melanins synthesized in nature from precursors such those described in the aforementioned patent, i.e. from aloin and related anthrones or from p-diphenols and/or hydroxyanilines.

The first oxidation steps of caffeic acid was studied by John M; Gumbinger HG and Winterhoff H Planta Med, 56(1):14-8 (1990); whilst polyphenoloxidase in relation to browning on fruits was studied by Macheix J.J. et al., in Crit. Rev. Food Sci. Nutr., 13(4):441-86 (1991) and Prabha T.N. et al.,. in Ind. J Bioch. Biophys., 13(6):402-5 (1981).

The extraction of vegetal melanins is disclosed in WO00/09616, wherein melanin polymers are obtained by extraction procedures from suitable plant material. However, the extraction route consists in an expensive and time-consuming multistep procedure, yielding small amounts of vegetal melanins, so that the product is not rendered available for large scale application.

Moreover, the extraction methods provide only the oligomers of vegetal melanins, as high molecular weight melanins are not extracted, due to their strong bounding within the vegetal matrix formed by the other vegetal biopolymers, and because of their low solubility in common solvents.

Finally, the extracted vegetal melanin may suffer a broad range of variability in their physico-chemical properties, due to the large variation of the both polymerization condition and the plant polyphenols composition, for example caused by the climatic variation along the year, the different cultivars, and the different grade of ripening.

US-A-5,384,116 discloses synthetic melanins made by combining dopachrome and an appropèriate enzyme or by incubating 5,6-dihydroxyindole-2-carboxylic acid alone or with 5,6-dihydroxyindole or with 3-amino-tyrosine, useful as sunscreen and tanning agents.

### BRIEF DESCRIPTION OF THE DRAWINGS

Some illustrative embodiments of the invention and examples of carrying out the invention, are described in detail hereinbelow, with reference to the accompanying drawings in which:
FIG. 1 is schematic diagram of the conversion of some representative plant monophenols into diphenols, the actual vegetal melanin monomers;
FIG. 2 is a schematic flow diagram illustrating the Raper-Mason pathway from main eumelanin precursors, whose condensing centers are highlighted by double arrows;
FIG. 3 to 6 are schematic view of UV-visible spectra of synthetic vegetal melanins according to the present invention.

### DETAILED DESCRIPTION OF THE INVENTION

It is commonly understood that products intended for use in industrial preparations must show constant parameters, be reproductible and free from contaminants.

We have now found out that synthetic vegetal melanins showing reproducible physical features may be conveniently produced in vitro.

We have also found out that high molecular weight synthetic vegetal melanins showing reproducible physical characteristics may be conveniently produced in vitro.

In fact, we have ascertain that in in vitro oxidative conditions, the ortho-diphenolic moiety of vegetal melanins precursors, such as the polyphenols are converted into semiquinones and quinones, thus making the diphenolic ring available to polymerize, either by self-condensation or copolymerization with other polyphenols and/or with eumelanin precursors.

We have ascertain that high molecular weight synthetic vegetal melanins having reproducible features may be produced starting from a given composition of suitable monomers submitted to controlled polymerization conditions.

Therefore, according to one of its aspects, the present invention concerns synthetic vegetal melanins, more specifically the synthetic vegetal melanins herein defined as omo- and ethero-polymers, obtained by in vitro polymerization of purified plant polyphenols, optionally with copolymers such as the eumelanin precursors, more particularly obtained by in vitro polymerization of at least one monomer unit (a), and optionally at least one compound (b), wherein:
(a) is a plant polyphenol; and
(b) is an eumelanin precursors; said synthetic vegetal melanin being characterized by a Red:Green:Blue (RGB) ratio comprised between 1 : 0.88 : 0.84 and 1 : 0.64 : 0.41.

Preferably the molecular weight of at least 80% of the synthetic vegetal melanins of the present invention is ≥ 0.8x10⁴ dalton.

The synthetic vegetal melanins of the present invention are characterized by reproducible physical characteristics, such as color, UV-visible spectra, and the antioxidant activity.

According to a preferred embodiment, the invention concerns synthetic vegetal melanins obtainable by polymerization of at least one plant polyphenol selected from
- formula (I): 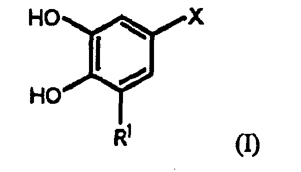
   wherein:
   - R¹: represents hydrogen, hydroxy, or methoxy group; and
   - X: represents a residue of formulas (II) or (III)

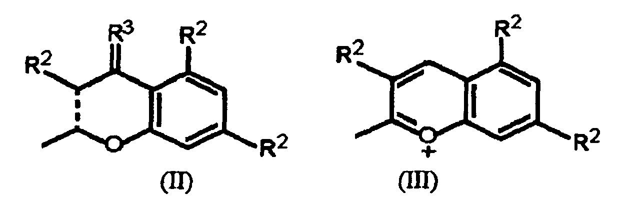

   wherein:
   - R²: represent, each independently, hydrogen, hydroxy, methoxy, or an O-glycosilated group;
   - R³: represents, one oxygen, two hydrogen atoms, or one hydrogen and one hydroxy group; and

   the phantom line between C₂-C₃ represents a double or a single bond,
   the phantom line between C₂-C₃ represents a double or a single bond,
- protocathechuic aldehyde;
- tannic acid;
- hydroxytyrosol, gallic acid and mixture thereof;

as well as salts, esters and solvates thereof.

In controlled oxidative conditions, the plant polyphenols of formula (I) are converted into the corresponding semiquinones and quinones, which afterward undergo either self-condensation or copolymerization with other plant polyphenols and/or with eumelanin precursors.

The synthetic vegetal melanins of the present invention, are thus primarily characterized by distinctive and reproducible physico-chemical features, such as the molecular weight, the color and their spectroscopic features, thanks to the control of the starting monomeric composition and of the polymerization conditions.

The main distinctive feature of the synthetic vegetal melanins are their color. In fact, all melanins exhibit a broad band of absorption within the UV-visible range, with a smooth decreasing pattern toward high wavelengths, with no detectable maxima. Therefore the UV-visible spectrum may not clearly identify any single melanin, whereas the color tones are clearly distinguished by a color analyzer. The color space dimension is evaluated by a three-points parameter, such as the Red, Green, Blue (RGB) ternary value. Moreover, when the RGB ternary value is expressed as R:G:B ratio, it univocally identify the melanin by its color characteristic, hence regardless of the concentration of the sample.

It is well known that the color intensity greatly influence the color perception, therefore a color definition will greatly differ according to the sample concentration. On the other side the R:G:B ratio of the synthetic vegetal melanins of the present invention is a costant and reproducible feature. Moreover, it was surprisingly found that the R:G:B ratio does not generally differs when the melanin is in ionic (salt) form, such as the sodium salt, or in non-ionic (acidic) form.

In that respect they clearly differ from the natural occurring vegetal melanins, which may vary in a broad range of chemical composition and/or molecular weight due to the seasonal and specimen variation of the concentration and composition of the plant polyphenols, as well as the polymerization conditions such as temperature, humidity, oxygen partial pressure on the cultivated land and so on.

The synthetic vegetal melanins of the present invention are thus obtained by an oxidation process, also called melanization process, starting from highly purified precursors in presence of atmospheric oxygen, to afford synthetic vegetal melanins of high molecular weight, substantially higher than 80%, as calculated using the elution times from a precalibrated molecular sieve HPLC column.

The synthetic vegetal melanins of the present invention, thus produced in presence of atmospheric oxygen from highly purified selected monomers, i.e. plant polyphenols and optionally eumelanin precursors, are characterized by distinctive photoabsorption patterns.

The in vitro ex-vivo antioxidant test carried out on synthetic vegetal melanins of the present invention exhibited reproducible antioxidant activities. Generally the inhibition of the metal-dependent oxidation (secondary oxidation) is higher than the radical scavanging activity (primary oxidation) in model system. This is due to the capacity to form polydentate complexes with the pro-oxidant metals, with significant inhibition of Fenton-like reactions.

The synthetic vegetal melanins of the present invention are further characterized by a large availability of colors, thus are particularly suitable for a variety of application in the health care industry.

The term "plant polyphenol " as used in the present invention designates those molecules bearing an ortho-dihydroxyphenolic moiety typically occurring as plant secondary metabolites.

Preferred plant polyphenols are those polyphenols of formula (I) above.

Common plant polyphenols may either have a polycyclic structure, such as flavonoids, or an open-ring diphenolic structure.

Illustrative example of flavonoids are flavonols such as quercetin and its glycosides, as the 3-rhamnoside (quercetrin) from many plants (e.g. horse chestnut) or the 3-rutinoside (rutin) from grape, tobacco and eucalyptus plants; flavons such as fisetin and fustin from Venice sumac; or mixture of flavonoids and benzophenones such as in morin and maclurin from old fustic (Morus tinctoria).

Further illustrative example of flavonoids are those contained in grape and in the peel of other edible fruits, thus containing flavanonols such as myricetin, flavones such as luteolin, flavandiols such as dihydroquercetin, as well as the flavanols. Grape contains also the anthocyanins and chalcones, which are the biosynthetic precursors of flavonoids and anthocyanins, the latter being slowly reverted to the corresponding chalcones when kept in neutral or slighly alcaline aqueous solution. Anthocyanins are the coloured matter of flowers and also occurring in coloured fruits and vegetables, including cyanidin and delphinidin, which may also be substituted as mono, di-saccharides, and position 3 may be acylated, e.g. with p-coumaric acid.

Further illustrative example of flavonoids are the oligomeric proanthocyanidins (OPC), precondensed oligomer of flavanols, i.e. (+)-catechin and (-)-epicatechin, which are commonly extracted from grape seed, pine bark, cocoa and other vegetal sources rich in flavan-3-ols in the free, glucosylated, esterified, or condensed forms. Galloylated catechin monomers are typically extracted from green tea or Catechu gambir, which contain (+)-gallocatechin (GC), (+)-gallocatechin gallate (GCG), (-)-epigallocatechin (EGC) and (-)-epigallocatechin gallate (EGCG).

Further polyphenols for our purposes may include those plant diphenols which are biogenetically correlated with flavonoid, e.g. the benzyndenpyranones such as brazilin from brazilwood and sappanwood, and haematoxylin from logwood; or the isoflavones such as glycitein from soybean.

Other plant polyphenols which may be used in the present invention are the open ring dihydroxyphenols.

Illustrative example of open ring dihydroxyphenols are hydroxytyrosol and its esters (oleuropein, verbascoside, etc.) found in olive fruit and leaves; protocathechuic acid found in most higher woody plants and in wheat; and protocathechuic aldehyde, found in vanilla, and other phytoalexins.

Further illustrative example of open ring dihydroxyphenol are gallic acid and its derivatives, which may be obtained from hydrolisis of plants rich in tannins, such as nutgalls. Tannins (alias tannic acid) are also classified as ellagitannins or gallotannins, the latter releasing gallic acid by hydrolisis treatment.

Plant polyphenols suitable for our purpose may occur either in the free form (aglycones) or as natural occurring esters and ethers (e.g. glycosides, gallates, and the like) as well as in oligomeric form (e.g. proanthocyanidins and viniferins).

Glycosides are the O-derivatives of the plant polyphenols in the plain form (aglycones), generally substituted in one or two hydroxy groups in the 3, 5 or 7 positions with naturally occurring mono- di- or trisaccarides, such as ruthoside, galactoside, rhamnoside, glucoside, sophoroside, rhamnoglucoside, and the like.

Plant polyphenols suitable for the present invention may be in form of titolated plant extracts with known chemical composition, such as obtained by standardized extraction methods from vegetal sources, either in solid form, e.g. dry extracts, or in liquid form, e.g. hydroalcoholic or as an alkaline solution.

In the present invention, plant polyphenols are purified compound, which are in the form of enriched fractions or even as pure compounds, either purified from natural sources or produced by chemical synthesis, so as to achieve synthetic vegetal melanins having the desired compositions, their physical features being therefore industrially reproductible. Another important technical advantage of using plant polyphenols as enriched fractions or even as pure compounds is that formation of foam during the polymerization reaction is thus reduced.

In a further embodiment of the present invention there are provided "mixed-type" synthetic vegetal melanins, thus produced by the oxidative copolymerization of plant polyphenols with eumelanin precursors.

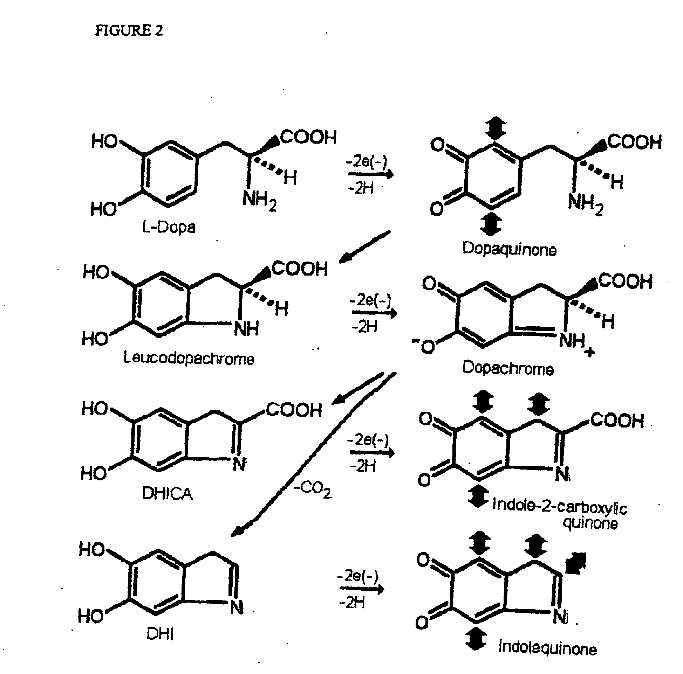

As shown in FIG. 2 by the Raper-Mason pathway (Physiol. Rev. 8. 245, 1928; J. Biol. Chem. 172, 83, 1948) the typical eumelanin precursors are L-dopa and its melagenetic intermediates: dopa-semiquinone, dopaquinone, leucodopachrome, 5,6-dihydroxyindole-2-carboxylic acid (DHICA), dopachrome, 5,6-dihydroxyindole (DHI), and indole-5,6-quinone.

L-dopa occurs in several plants, such as Mucuna prurienses and Vicia Faba; DHICA and DHI occurring in plants such as Rhaphidophora korthalsii; 3,4-dihydroxyphenylethylamine (dopamine) is occurring in vegetables and fruits, such as in the peel of banana or potatoes; pyrocatechol present in some vegetable such as garlic; and pyrogallol found in geranium and Hypericum p.

Preferred eumelanin precursor for the present invention are dopamine and L-dopa, both being partially polymerized "as such" and partially in situ converted into typical eumelanin indolic precursors by contact with oxygen, as per Raper-Mason pathway.

The synthetic vegetal melanins of the present invention can be prepared from plant polyphenols, with or without eumelanin precursors, by a number of chemical or enzymatic polymerization procedures.

A preferred method of producing synthetic vegetal melanins is the chemical method, i.e. bubbling air or oxygen through an alkaline aqueous solution of the monomers at pH 10 or above (e.g. by 1 N sodium hydroxide or 2-3 N ammonia) during 12 to 48 hours (preferably 24 hours), at a temperature ranging from 10 to 90°C, preferably in the range of temperature from 20 to 30°C. The alkaline aqueous solution may comprise catalytic amounts of pro-oxidant metals, such as Cu⁺⁺, Fe⁺⁺, Ni⁺⁺, or Co⁺⁺ solutions, for example at 5 to 20 mM concentration.

The amount of alkali depends on the chosen plant polyphenol, i.e. the strength and quantity of ionizable hydrogen atoms to be salified in order to achieve the full solubilization of the starting monomers. For strong alkali such as NaOH or KOH the optimum ratio with the melanin precursors is around 3:1 equivalents, whereas for weak alkali such as ammonia the optimum ratio with is around 10:1.

A fast polymerization procedure may also be carried out by using chemical oxidizing agents, such as hydrogen peroxide, hydrogen iodide, ammonium persulfate, potassium permanganate, or magnesium perchlorate. If such strong oxidants are employed, the synthetic vegetal melanin precursors may also be present as monohydroxylated (phenolic) form, and thereafter converted in situ to the corresponding diphenol compounds (the actual melanin monomers) in a "one-pot" oxidative polymerization reaction.

A further preferred method of producing synthetic vegetal melanins of the present invention is the enzymatic synthesis by suitable melanin-forming enzymes, such as tyrosinases, polyphenoloxidases (catechol oxidases), phenolases, (phenoloxidases), peroxidases, laccases, lypoxygenase, and mixture thereof.

The enzymatic process makes use of a stream of air or oxygen through a buffered aqueous solution of plant polyphenols and/or eumelanin precurosors during 12 to 48 hours or more, preferably at controlled temperature in presence of a suitable enzyme. In this case, the synthetic vegetal melanin will be produced in low concentration, due to their low solubility in water of the plant polyphenols, present in their neutral (non-ionic) form.

Suitable enzymes for the synthesis of the synthetic vegetal melanin of the present invention may be obtained from several melanin-producing biological cells or tissues, such as:
(1) extracts of animal skins, e.g. horses, cattle, sheep, pigs, or any other such mammalian source; extracts of skins of fish, amphibia, reptiles, birds;
(2) extracts of melanin-producing vegetal source, e.g. mushrooms, potatoes, bananas;
(3) extracts of melanin-producing invertebrate organisms, e.g. worms, arthropods;
(4) extracts of melanin producing microorganism, e.g. bacteria and protozoans;
(5) extracts of any organisms in which genes for enzymes producing soluble melanin have been genetically cloned; and
(6) culture media of any organisms or cells which secrete enzymes for producing soluble melanin-such organisms or cells may or may not express cloned genes for the enzymes.

Some of the above mentioned enzymes are commercially available and the others can be prepared by known techniques.

Furthermore, the enzymes capable of actively synthesizing melanin may not necessarily have to be isolated from the organism producing thereof.

Particularly when the enzyme applied retaining a significant hydroxylating activity, the monomers of synthetic vegetal melanin may be formed in situ from the corresponding monophenols (the "pre-monomers"), as in the case of the chemical synthesis in presence of chemical oxidizing agents.

Illustrative examples of pre-monomers of synthetic vegetal melanins include dihydrokaempferol, armadendrin, apigenin, p-hydroxybenzaldehyde, PHBA, tyrosol, p-coumaric acid, kaempferol, pelargonin, and genistein.

Illustrative examples of pre-comonomers of synthetic vegetal melanins include L-tyrosine, tyramine, and 5-hydroxy-indole.

The polymerization may be carried out in a standard fashion, i.e. by introducing all the monomers at once, as well as by separate or sequential polymerization of a portion of monomers, as to provide melanin "block copolymers".

The synthetic vegetal melanins may be used for their application either as the native solution or in solid form, the latter obtained by a number of precipitation procedures known to those skilled in the art, for example by addition of an organic or mineral acid, e.g. HCl, H₂SO₄, acetic acid, glycolic acid, or L-tartaric acid; or by addition of water-soluble organic solvents, for example 2 volumes of acetone or ethanol; or by addition of divalent or trivalent metal salts, for example solution of ZnCl₂, MgCl₂, AlCl₃, or CaCl₂, thus forming insoluble metal complexes; or by mixed techniques.

Moreover, the synthetic vegetal melanins may be stabilized by stopping the polimerization by the addition of boric acid or salt thereof, which are added to the final reaction mixture to prevent further increase of the molecular weight.

Further modification to the structure of the synthetic vegetal melanin may be applied in order to alter their chemical and physical properties.

Esters of synthetic vegetal melanins can be prepared by standard methods of acylation, such as by the reaction of the melanin with acid chlorides or acid anhydrides in the presence of base. Of particular utility is the preparation by reaction with acid anhydrides in pyridine as utilized for the acylation of certain other lower molecular weight flavonoids by Thompson, et al., JCS, 1387 (1972), Fletcher, et al., JCS, 1628 (1977) and Hemingway, JCS, 1299 (1982).

Ethers can be prepared by standard methods of etherification such as by reaction of the synthetic vegetal melanin with alkyl halides and alkyl tosylates in bases. Methyl ethers are readily prepared by the use of diazomethane as utilized in the etherification of certain other low molecular weight flavonoids by Thompson, JCS, 1387 (1972), Hemingway, JCS, 1299 (1982).

The synthetic vegetal melanins may be isolated and/or purified by filtration, ultrafiltration, fractional sedimentation, freeze-drying, spray-drying, dialysis, centrifugation or combination thereof.

Alternatively, the synthetic vegetal melanins of the invention may be directly used as aqueous solutions, then complexed or compounded with suitable ingredients during the preparation of cosmetic or pharmaceutical compositions. When the synthetic vegetal melanins are used in a soluble or suspended form in a fluid preparation, they are preferably associated with at least one part in weight of a suitable surfactant, such as cationic, anionic, amphoteric and non-ionic surfactants, in order facilitate the dispersion and stabilize the biopolymer during storage.

The synthetic vegetal melanins of the present invention may be used in pharmaceutical and cosmetic preparations to provide protection against oxidative stress, due to the presence of the highly active vegetal moiety within the polymeric network.

In addition, the synthetic vegetal melanins of the present invention can be produced in a large variety of colors, thus being a valuable tool for producing cosmetic compositions for facial make-up and hair dyes, as well as in tanning, anti-sun, moisturizing-protecting compositions and toiletry preparations.

It is a further object of the present invention to provide cosmetic compositions comprising the aforementioned synthetic vegetal melanins.

The cosmetic composition according to the invention may further comprise any cosmetically acceptable ingredients, for example those included in the INCI list drawn by the European Cosmetic Toiletry and Perfumery Association (COLIPA) and issued in 96/335/EC "Annex to Commission Decision of 8 May 1996" and further modifications.

Preferred cosmetic ingredient to be associated at the synthetic vegetal melanins in fluid cosmetic formulations are the surfactants, thus including anionic, cationic, nonionic and zwitterionic surfactants, either with medium-low molecular weight or as oligopolymer and polymer containing a polarized moiety within a predominantly hydrophobic structure.

Among surfactants are particularly preferred those bearing a positive charge, thus capable of forming a ion pair with the substantially negatively charged synthetic vegetal melanins, therefore stabilized and kept in a suspended/solubilized form.

More preferably, the synthetic vegetal melanins in solution or in a precipitated form are premixed with one or more surfactant (e.g. soybean lecithin and mono- di-glycerides) prior the mixing with the rest of the formulation, to produce a stable color, an homogeneous appearance and enhancing stability and shelf life of the cosmetic formulations containing thereof.

The cosmetic composition of the invention can be formulated as a lotion, a fluid cream, a cream or a gel. The composition can be packaged in a suitable container to suit its viscosity and intended use by the consumer. For example, a lotion or fluid cream can be packaged in a bottle or a roll-ball applicator, or a capsule, or a propellant-driven aerosol device or a container fitted with a pump suitable for finger operation. When the composition is a cream, it can simply be stored in a non-deformable bottle or squeeze container, such as a tube or a lidded jar.

The invention also refers to pharmaceutical and/or nutritional compositions comprising synthetic vegetal melanins, which may provide anti-inflammatory and immunomodulation activities by the oral intake. The synthetic vegetal melanins may be also used in oral compositions for their coloring properties, further associated with an antioxidant behaviour to increase the shelf-life of the edible products comprising thereof.

The oral compositions comprising synthetic vegetal melanins to be administered by the oral route in solid form such as sachets or soft gelatin capsules, fast or slow-release tablets, or in liquid or emulsified forms, and may also include further active ingredients and acceptable excipients, for examples those described in "Remington's Pharmaceutical Sciences Handbook", Mack Pub. Co, USA.

According to another of its aspects, the invention relates to a method for treating and/or preventing diseases due to exposure to radiations or to oxidative environment as well as in inflammatory diseases or immunitary unbalance, which - comprises administering to a subject an effective amount of at least a synthetic vegetal melanin of the invention.

Furthermore, the synthetic vegetal melanins may be used as food ingredient (additive) for the manufacturing of health food, dairy products and canned food, as well as in beverages such as soft drinks, syrups and fruit juice.

The following examples are preferred features of the invention but are not intended to limit it in any way.

### EXAMPLES

### Method A - Chemical synthesis of synthetic vegetal melanins

A weighted amount of a total monomers, calculated from the actual polyphenolic content of the starting material, is charged into a flat-bottom conical flask (500 ml) and solubilized in 200 ml of sodium hydroxide 1 N (the amount of sodium hydroxyde shall undergo minor variation according to the Kₐ and the number of acid centers whithin the molecule of each plant polyphenol, i.e. according to the quantity and quality of ionizable hydrogen atom to be salified in order to achieve the full solubilization of the starting monomers).

The flask, placed in a thermostatic bath at 24°C, is equipped with an air pump (Silent Air®, manufactured by Renn-Plax, Inc. in Taiwan R.O.C.) connected by a flexible hose to a disperser, which is placed at the bottom of the flask. The air is forced through the disperser and finely divided throughout the alkaline solution, which, for the oxidative polymerization, is kept under air bubbling for a time ranging from 12 to 48 hours at a temperature of 24°C. The water lost due to the evaporation caused by the stream of air was refilled from time to time in the reaction vessel.

The reaction mixture readily develops colors, as vegetal melanins absorb widely throughout the ultraviolet and visible spectra (e.g., 220 to 700 millimicrons), the final color of the melanin solution varying according to the monomer composition and, at less extent, to the reaction time.

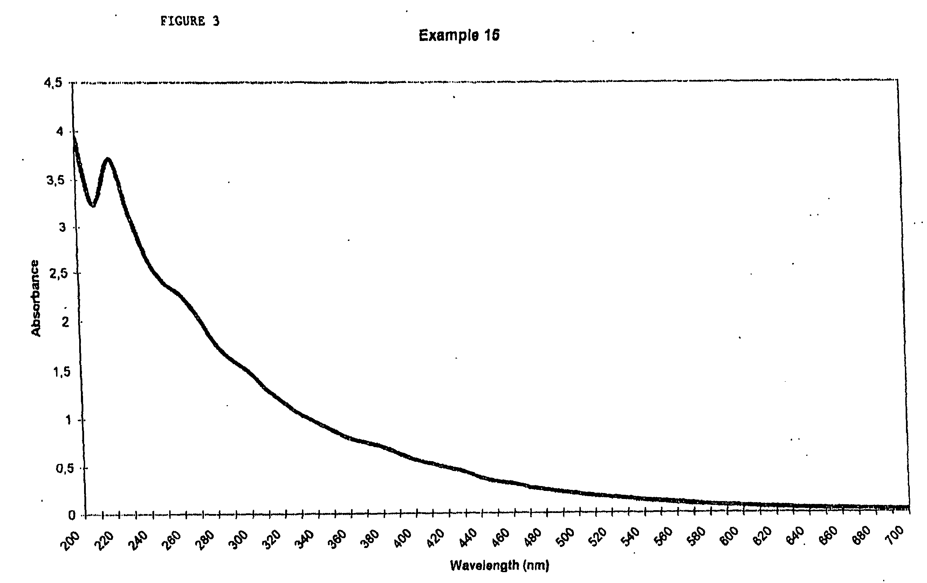

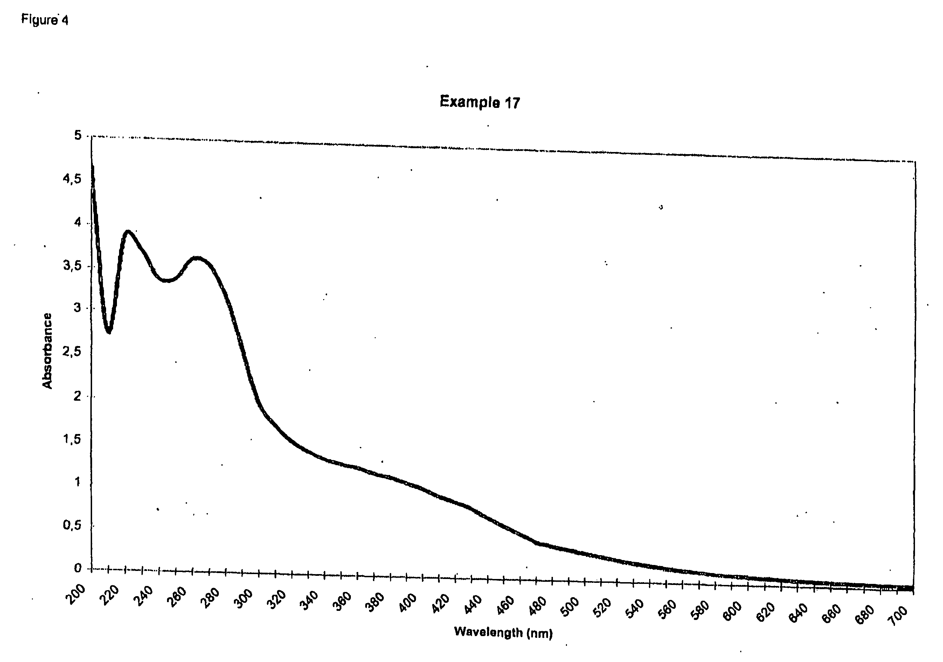

Absorbance of vegetal melanins was measured by spectrophotometric absorbance of a solution obtained by dissolving 4 mg of synthetic vegetal melanin in 100 ml of 0.1 N NaOH. The UV visible absorption spectrum was recorded on a spectrophotometer between the wavelength of 200-700 nm in 1 cm path length cuvette. The representative spectra (200-500 nm) of a selected number of vegetal melanins are shown in FIG 3, 4.

The aforementioned procedure were modified to provide further vegetal melanins, which are modified from the original omo- or etheropolymer under the circumstances described herewithafter.

### Modification applicable to the method A

### i) The use of pro-oxidant transition metal ions, such as Cu⁺⁺, Ni⁺⁺, Co⁺⁺, Fe⁺⁺ to enhance oxidative polimerization.

When pro-oxidant metal ions are added to the reaction mixture, a darker tone is generally obtained, the effect being low for vegetal melanins comprised of flavonoid-type plant polyphenols, higher in the presence of plant polyphenols with open catechic structure and even more pronounced with mixed-type vegetal melanin, e.g. with high L-Dopa to plant polyphenols ratio.

In the next Examples, 1,25 mM Cu⁺⁺ solution is formed by solubilizing 20 mg CuSO₄*2H₂O into 100 ml of the reaction mixture.

### ii) The use of weak bases instead of NaOH or KOH solutions.

The vegetal melanins display a generally darker tone in presence of ammonia or other ammonium bases (e.g. triethanolamine) as alkalifying agent. Noteworthy, in ammonia solution there is low, if any, catalytic activity of pro-oxidant metals salt such as Cu⁺⁺, which shall be due to the partial inactivation by the Cu-ammonium complex.

In the next Examples, ammonia 2.5 N is used as alkalifying medium instead of sodium hydroxide.

### iii) The use of chemical oxidant, organic or inorganic peroxides.

The strong oxidating agents, such as persulfates, KMnO₄ or hydrogen peroxide, may be added to the reaction mixture to produce synthetic vegetal melanins. Moreover, these chemicals are capable of converting the pre-monomers in the actual melanin precursors, so that the corresponding monophenols can be used instead of the diphenolic monomers, and converted in situ to the actual precursors by the oxidant agent, finally being polymerized by the combined activity of ambient oxygen and chemical oxidation.

If 40 ml/l of H₂O₂ 20 vol is added dropwise to the polymerization mixture during the reaction additionally a pale color is generally obtained. The UV spectrum of bleached vegetal melanins shows a absorption flattening at higher wavelength. The bleaching effect is comparatively higher on vegetal melanin with low plant polyphenols to eumelanin precursor ratio.

In the next Examples, ammonium persulfate in 2:1 molar ratio as regard to the total pre-monomer content is added to the reaction mixture under high stirring to provide oxygen circulation, for 24 hours. At the end of reaction a brown-red vegetal melanin is obtained.

### Method B1 - Enzymatic synthesis of the vegetal melanins by laccase

A weighed amount of a total plant polyphenols, calculated from the actual monomer content of the starting material, is charged into a flat-bottom conical flask (250 ml) and solubilized in 100 ml of a solution 20 mg of laccase at 0.8 LAMU/mg (Denilase® II S, Novo Nordisk A/S, Bagsvaerd, Denmark) in phosphate buffer 50 mM at pH 6,5. The solution is kept at a temperature of 37°C under high stirring by a magnetic bar, so to force hair circulation for 24 hours. At the end of reaction insoluble, colored vegetal melanins are obtained.

### Method B2 - Enzymatic synthesis of the vegetal melanins by tyrosinase

A weighed amount of a total plant polyphenols, calculated from the actual monomer content of the starting material, is charged into a flat-bottom conical flask (250 ml) and solubilized with 20 mg of mushroom tyrosinase at 2200 U/mg polyphenoloxidase (Sigma, St.Louis, USA) in 800 ml phosphate buffer at pH 6,5 under stirring. The solution is kept at a temperature of 24°C under high stirring to provide brown-black vegetal melanins.

### Modification appliable to both Method A and Bs

### iv) The use of boric acid or its soluble salts to stop post-polymerization pathways.

When borate and melanins are contacted in solution, a bidentate link of the orthoboric structure and the ortho-quinoid moiety of the biopolymer to provide a yclic ester is postulated to occur, thus preventing the further darkening and post-polymerization of the synthetic vegetal melanins.

In the next Examples, boric acid is added 30 minutes before the end of the reaction at about 1:2 molar ratio with respect to the total amount of the starting vegetal monomer units.

### v) The use of anti-foaming agent during oxidative polymerization.

The use of anti-foaming agents at low concentration is needed to avoid excess foam formation during water injection for plant polyphenols from medium purity extracts, thus with a residual content of proteins, saponins and other naturally occurring, foam-making substances.

In the next Examples, Dow Corning® 2210 is added at 5-40 ppm into the reaction mixture.

### C) Precipitation of the synthetic vegetal melanins

The vegetal melanins may be obtained in solid form after precipitation by the addition of acid, of water-soluble organic solvents, or solution of di- or trivalent metal salts, and combination thereof.

In the next Examples, precipitation of vegetal melanins was carried out by a addition of one equivalent ZnCl₂ 1M in water, then the reaction mixture was heated at 60°C for 5 minutes and cooled to ambient conditions. The slurry is washed with demineralized water, filtrated, then dried in nitrogen stream, and the dry solid is grinded.

### Materials - Monomers and precursors of the synthetic vegetal melanins

- Quercetin 2H₂O; MW = 338; content 99%; from Fluka Chemie AG (Buchs, CH).
- Rutin 3H₂O; MW = 664.5; content 96%; from Austin Ch. Co. (Buffalo Grove, IL, USA).
- Anthocyanins with grape flavonoids (AGF) (4:1 mol/mol), average MW = 338.7 (calculated as delphinidin); 20% solution; source grape pomace; from Enocianina Fornaciari Srl (Reggio Emilia, Italy).
- Grape seed oligomeric proanthocyandins (GS-OPC); average MW = 290 (calculated as catechin monomer); content ≥ 90%; from Centroflora Ltd (Sao Paulo, Brazil).
- Green tea polyphenols (GTP); average MW = 316 (calculated as gallocatechin); content 95%; from Xinguang Ind. Products (Chengdu, Sichuan, P.R. of China).
- Fustic extract, 40% solution with morin and myricetin, average MW=302 (calculated as morin); from CE Roeper GmbH (Hamburg, Germany).
- Gallic acid H₂O; MW = 188; content ≥ 98%; from AffaChem (N. C. Islip, NY, USA).
- Protocatechaldehyde (PA1);MW = 138; content 99%; from Ubichem Plc (Eastleigh, UK).
- Tannic acid; average MW = 634 (calculated as corilagin); content ≥ 90%; from Sigma-Aldrich Srl (Milan, Italy).
- Naringin; PM = 580.5; content 95%; from Freeman Industries Inc. (New York, NY, USA).
- L-Dopa; content ≥ 99%; MW = 197; content 99%; from Taiye Co. (Kunshan, P.R. of China).
- Dopamine; content ≥ 99%; MW = 153; content 99%; from Recordati Srl (Milan, Italy).
- Pytocatechol; content ≥ 99%; PM = 110; content 99%; from Borregard SpA (Ravenna, Italy).
- L-Tyrosin; content ≥ 99%; MW = 181; from Diamalt GmbH (Munich. Germany).

### Examples 1-15 - Synthetic vegetal melanins

Following the standard procedure of Methods A and B1 or B2 and further modifications (from i to v), 100 ml of the solution of monomers of pure plant polyphenols, as shown in Table I, or of a mixed solution of monomers of pure plant polyphenols and eumelanin precursors, as shown in Table II, were submitted to oxidative polymerization by dispersed air to provide synthetic vegetal melanins of different reproducible colors.

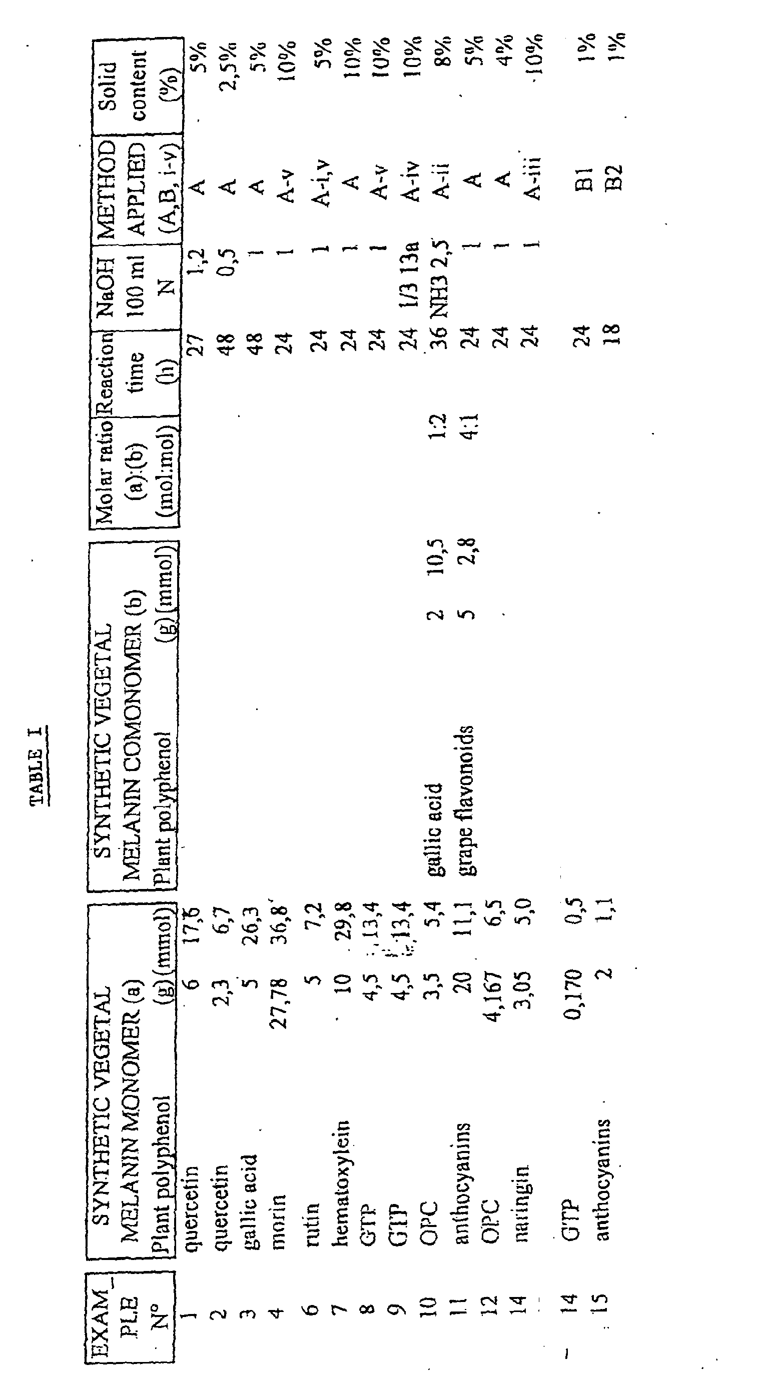

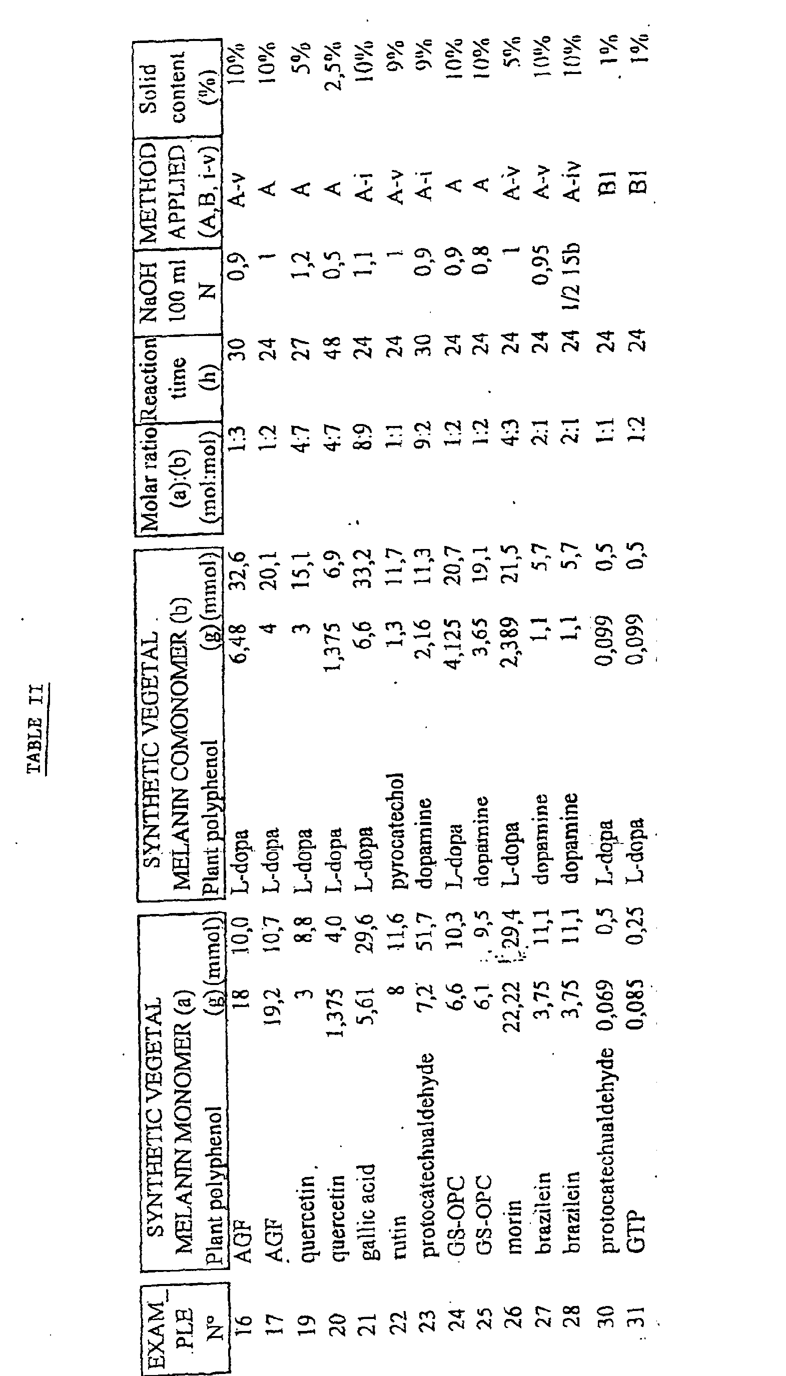

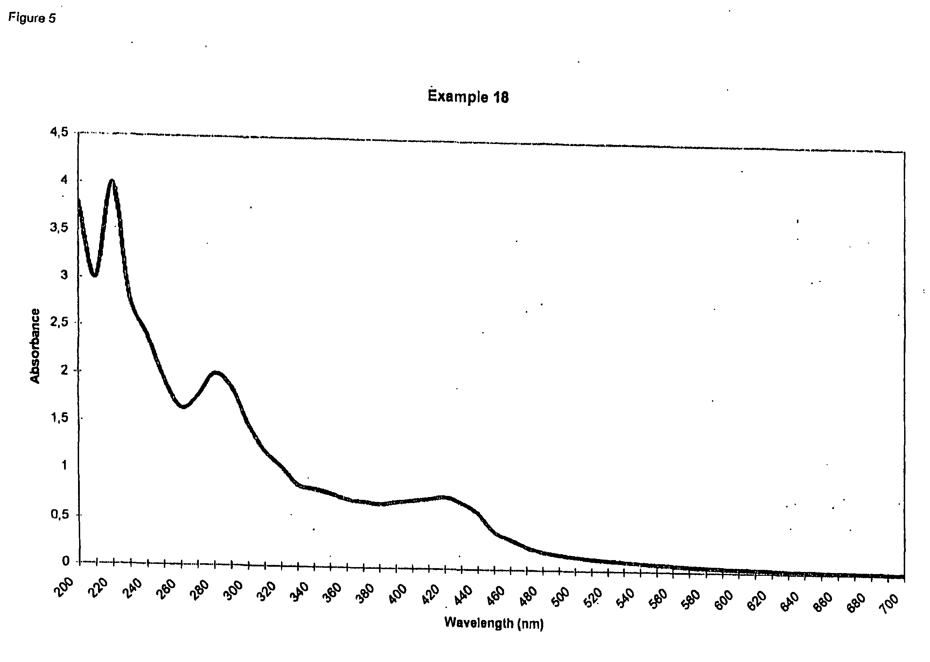

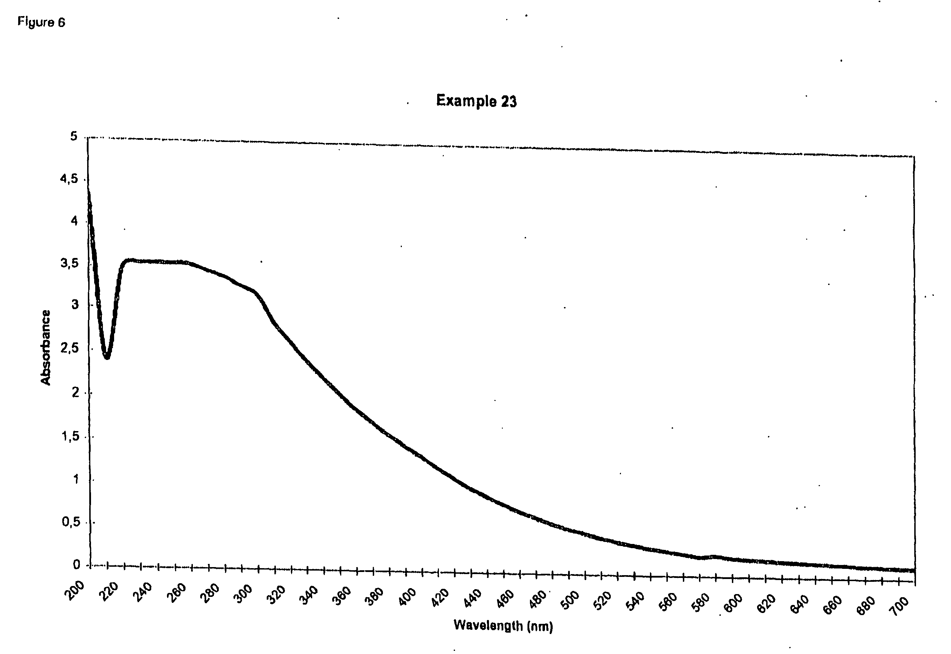

Each Example was repeated at least twice in the same operative conditions, providing synthetic vegetal melanins which exhibit reproductible physical features, such as color, MW distribution and the UV-spectra, whose representative examples are shown in FIG. 3 to 6.

The color of the synthetic vegetal melanins of Example 1-32 were analyzed by according a standardized procedure based on scanning of a small quantity of sample adsorbed onto a white paper and the analysis of the color composition.

In brief, a quantity of 0.05-0.2 ml of the solution were deposited on a filter paper (Whatmann N.3), thereafter the spot were dried at room temperature. For the synthetic vegetal melanin obtained by oxidative polymerization in alkaline solution, a spot were treated with approximately 0.1 ml of HCl 1N to obtain the corresponding non-ionic form.

The spots were scanned with HP ScannJet 4300C® Pro (Hewlett Packard) set in a fixed mode for input, aquisition as 16.7 million colors photo, with an output set at customized resolution (x20), resolution area of 239 units, shadows at 6 units and medium tones at 2.2 units.

The spots were then analyzed in different point by the software WhatColor v3.21e by Hikaru Kakahara (Japan) with the dither scope set at 8 surrounding pixels for the analyze of the average color of the spot of vegetal melanins by chemical synthesis, where those by enzymatic synthesis where analyzed with a single pixel scope.

The value was given in decimal figure with the RGB composition of the 3 main colors, namely Red, Green and Blue, as shown in Table III and Table IV.

The R:G:B ratio are thus not depending on the concentration of the melanin and are thus useful for the evaluation of both reproducibily and for applicative purposes.

Example 1-32 were evaluated with the afore mentioned method to provide consistent color ranging from the more yellowish tones to the reddish or scarlet browns, with a number of neutral and graysh brown tones in the middle; as shown in Table III and Table IV.

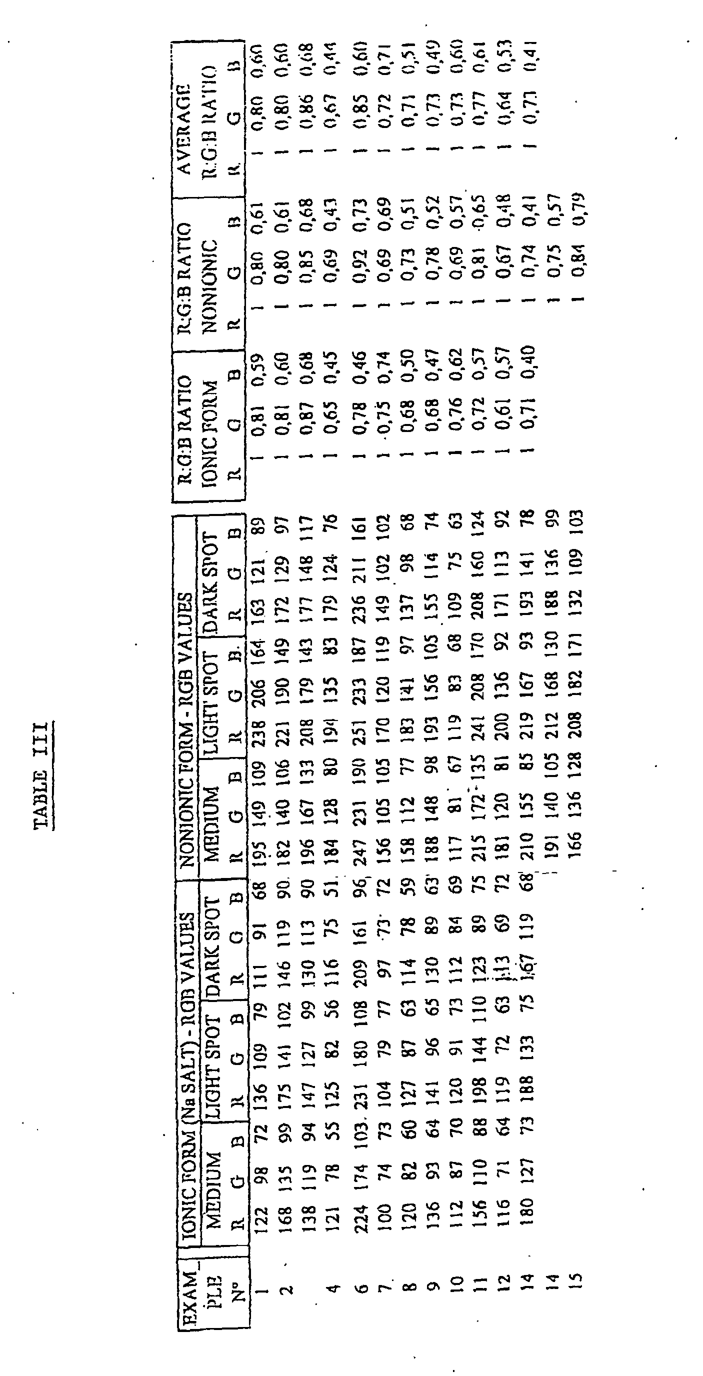

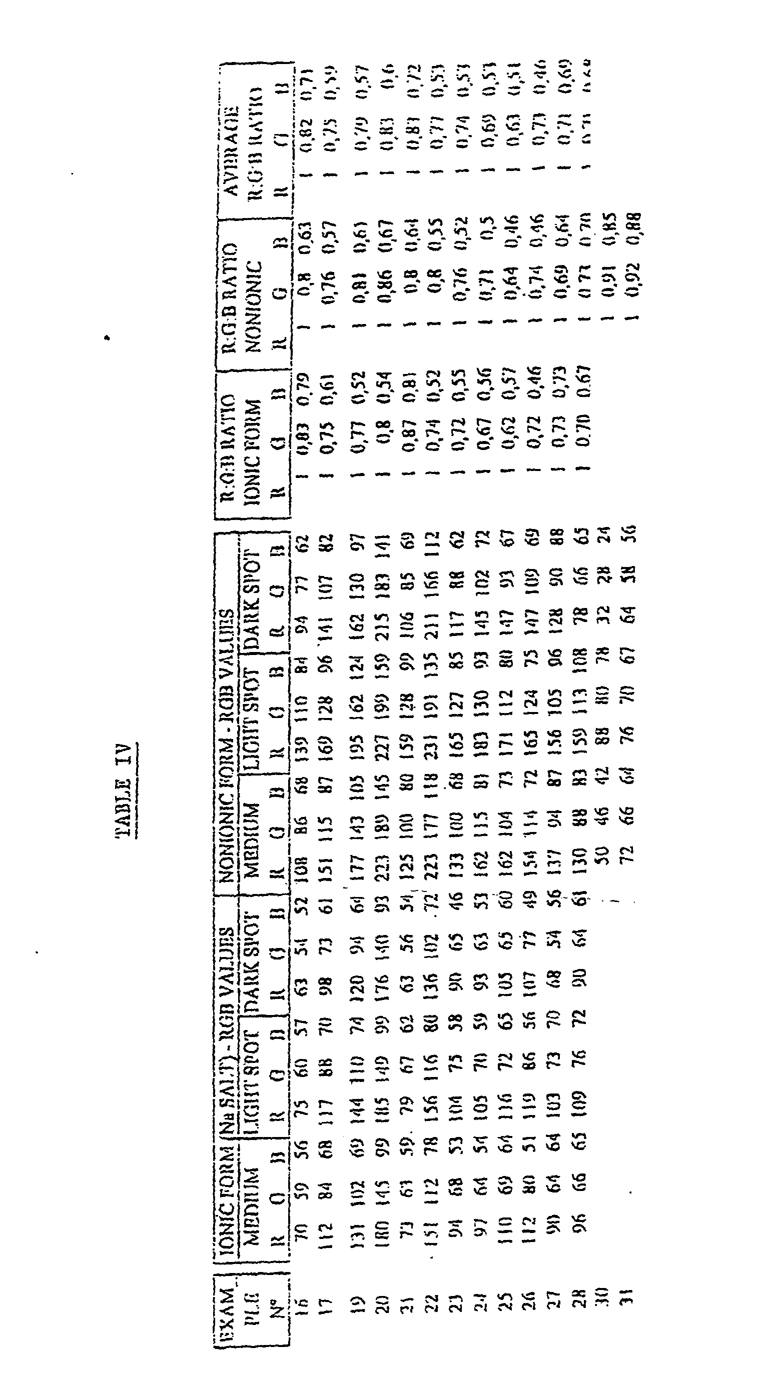

### Biological Example 1 - Inhibition of direct lipoperoxidation on LDL

The inhibition activity of the synthetic vegetal melanins has been carried out according to Visioli F. and Galli C. "Evaluating oxidation processes in relation to cardiovascular disease: a current review of oxidant/antioxidant methodology." in Nutr. Metab. Cardiovasc. Dis. 7: 459-466 (1997).

Human LDL (d=1.021-1.063) were isolated by sequential ultracentrifugation from plasma obtained from healthy, normolipidemic volunteers. Before initiation of the experiments, LDL samples were desalted by size-exclusion chromatography and their protein content was determined according to Lowry, O., et al., J. Biol. Chem., 1951, 193, 265 (1951).

Then 5 mg of the vegetal melanins were added and oxidation started by the addition of the free radical generator 2,2'-azo-bis(2-amidinopropane) dihydrochloride (AAPH) at concentrations of 5 mM. Incubation were carried out at 37 °C in a shaking bath and aliquots were withdrawn at different times for the analysis of the tiobarbituric acid reacting substance (TBARS) as oxidation marker, as shown in Table A.

**TABLE A Inhibition of LDL oxidation from 5 mM AAPH by vegetal melanins**

| Vegetal melanin | nMol TBARS/ mg LDL | Inhibition (%) |
|---|---|---|
| Control (no melanin) | 32.54 | 0 |
| of the Example 16 | 21.35 | 34% |
| of the Example 17 | 25.23 | 22% |
| of the Example 1 | 30.22 | 7% |
| of the Example 19 | 30.14 | 7% |
| of the Example 8 | 21.87 | 33% |
| of the Example 31 | 22.01 | 23% |

These data shown that the radical quenching activity is higher for the galloylated vegetal melanin of the Example 8 s well as in the "mixed type" vegetal melanin, thus which contain eumelanin precursors and open-ring polyphenols, either obtained by chemical or enzymatic synthesis, as for Example 16, 17 and 31. The lower value are reached by the presence of the unstable flavonol moiety from quercetin, as in Examples I and 19.

### Biological Example 2 - Dose-dependent inhibition of direct lipoperoxidation of LDL

The test was carried out as described in the Biological Example 2 but using the vegetal melanin of Example 16 at different concentrations. Results are listed in Table B.

**TABLE B**

| Concentration (ppm) | nmol TBARS/ mg LDL | Inhibition (%) |
|---|---|---|
| 0 | 7.05 | - |
| 1 ppm | 4.83 | 31% |
| 5 ppm | 3.57 | 49% |
| 10 ppm | 2.42 | 66% |
| 20 ppm | 1.92 | 73% |

As it can be noted, the radical scavenging activity (primary antioxidant inhibition) of the vegetal melanin follows an approximately first order reaction, with a significantly high decrease of the lipoperoxidation behavior for small increase of the vegetal melanin.

### Biological Example 3 - Inhibition of metal-dependent peroxidation of LDL

The test was carried out according Visioli F. and Galli C. (ibid): LDL were separated by sequential ultracentrifugation from human plasma and the samples were prepared by diluting LDL in PBS at a final concentration of 200 ug protein/ml, to a final concentration of 200 ug protein/ml. The probes were thereby pre-incubated for 30 minutes at 37°C with 5 mg of the vegetal melanins under investigation, then copper sulfate (5 uM, final concentration) was added to start the oxidation, which was carried out at 37°C in a shaking bath. At the end of the incubation period, samples were withdrawn for the quantitation of the tiobarbituric acid reacting substances (TBARS). Results are listed in Table C.

**TABLE C**

| Vegetal melanin | nMol TBARS/ mg LDL | Inhibition (%) |
|---|---|---|
| Control (no melanin) | 58.97 | 0 |
| of the Example 16 | 11.22 | 81% |
| of the Example 17 | 22.33 | 62% |
| of the Example 1 | 45.59 | 23% |
| of the Example 19 | 47.54 | 19% |
| of the Example 8 | 12.93 | 78% |
| of the Example 31 | 45.10 | 24% |

The aforelisted data exhibit a high correlation between the potential chelating activity and the structure of vegetal melanin Thus, the dictating behaviour is significantly high in all vegetal melanins but those formed with quercetin among starting monomers, still retaining an appreciable secondary antioxidant activity.

### Biological Example 4 - Dose-dependent inhibition of metal-dependent lipoperoxidation

The test was carried out as described in the Biological Example 3 but using the vegetal melanin of Example 16 at different concentrations. Results are listed in Table D.

**TABLE D**

| Concentration (ppm) | nMol TBARS/ mg LDL | Inhibition (%) |
|---|---|---|
| 0 | 10.15 | - |
| 1 ppm | 8.87 | 13% |
| 5 ppm | 4.91 | 52% |
| 10 ppm | 1.39 | 86% |
| 20 ppm | 1.43 | 86% |

The figures show that the inhibition by vegetal melanin of the metal-induced peroxidation does follow a steep slope, with almost complete deactivation already at 10 ppm. Nateworthy, this antioxidant activity is higher than most plant polyphenols, i.e. the vegetal melanin monomers. Not surprisingly, the concentration of 20 ppm do not offer any further protection, as the copper ions are already fully chelated by a polydentate complex with the vegetal melanin, thus any Fenton-like activity is therefrom inhibited.

### Applicative Examples 1-3 - Foundation

100 g of each foundations contain:

| | Appl. Ex. 1 | Appl. Ex. 2 | Appl. Ex. 3 |
|---|---|---|---|
| Triethanolamine stearate | 2.5 g | 2.5 g | 2.5 g |
| Glycerol mono- e distearate | 0.4 g | 0.4 g | 0.4 g |
| Magnesium silicate | 1.9 g | 1.9 g | 1.9 g |
| Pigment formed with the vegetal melanin of Example 13 (*) | 14.0 g | 10.0 g | 3.7 g |
| Pigment formed with the vegetal melanin of Example 16 (*) | 2.9 g | 8.0 g | - |
| Pigment formed with the vegetal melanin of Example28 (*) | - | 2,1 g | 15.0 g |
| Titanium dioxide (rutile) | 10.0 g | 10.0 g | 10.0 g |
| Miscela di PEG-6 e PEG-32 | 9.0 g | 9.0 g | 9.0 g |
| Nylon micronized | 9.0 g | 9.0 g | 9.0 g |
| Cyclomethycone | 13.0 g | 13.0 g | 13.0 g |
| Propylene glycol | 5.0 g | 5.0 g | 5.0 g |
| Glycerine | 4.5 g | 4.5 g | 4.5 g |
| Preservatives | 0.5 g | 0.5 g | 0.5 g |
| Deionized water | qb to 100 g | qb to 100 g | qb to 100 g |

| | | | |
|---|---|---|---|
| (*) In 100 ml of the solution of the vegetal melanin are suspended 13 g of alumina, the slurry is stirred and added with a solution of AlCl3 1M until pH 6-7. The slurry is then washed with demineralized water, filtrated, then dried in nitrogen stream, and the pigment is finally grinded. | | | |

The fatty phase containing the oils and stearic acid and the aqueous phase containing triethanolamine are separately heated to 80 °C. The mixture is emulsified at 80°C and cooled slowly. During the cooling, the mixture of pigments, previously ground in propylene glycol and cyclomethicone are added.

### Applicative Examples 4-6 - Preparation of a suntanning lotion

100 g of each suntanning lotions contain:

| | Appl. Ex. 4 | Appl. Ex. 5 | Appl. Ex. 6 |
|---|---|---|---|
| EO-21-stearyl ether (ICI G-1800) | 5.0 g | 5.0 g | 5.0 g |
| Isopropyl myristate | 10.0 g | 10.0 g | 10.0 g |
| Vegetal melanin solution of Example 4 | 1.0 g | 1.0 g | 1.0 g |
| Vegetal melanin solution of Example 6 | 1.0 g | 1.0 g | 1.0 g |
| Vegetal melanin solution of Example 14 | 1.0 g | 1.0 g | 1.0 g |
| Oleyl alcohol | 2.0 g | 2.0 g | 2.0 g |
| 2-Hydroxy-3,3,5-trimethylhexyl ester of benzoic acid | 4.0 g | 4.0 g | 4.0 g |
| BHA | 0.05 g | 0.05 g | 0.05 g |
| Perfume | 0.3 g | 0.3 g | 0.3 g |
| Distilled water | q.b. to 100 g | q.b. to 100 g | q.b. to 100 g |

The mixture is heated to 70°C and preheated distilled water, and the vegetal melanin is added thereto. The resultant mixture is stirred and allowed to cool to room temperature.

### Applicative Example 6 and Comparative Example 1 - Protective-tanning creams with test of suppression of UV-induced erythema

100 g of each emulsions contains:

| | Appl. Ex. 6 | Comp. Ex. 1 |
|---|---|---|
| Cetostearyl alcohol | 3.0 g | 3.0 g |
| Oleic polyoxyethylen glyceride | 5.0 g | 5.0 g |
| Gliceryl monostearate | 4.0 g | 4.0 g |
| 2-Ethylexyl cocoate | 2.0 g | 2.0 g |
| Tocopheryl acetate | 0.5 g | 0.5 g |
| Ascorbyl palmitate | 0.1 g | 0.1 g |
| Phospholipids (soy lecithin 63%) | 3.0 g | 3.0 g |
| Vegetal melanin solution of Example 25 | 3.0 g | - |
| Caramel solution 10% w/v in NaOH 1N | - | 3.0 g |
| Preservatives | 0.3 g | 0.3 g |
| Deionized water | qb to 100 g | qb to 100 g |

The mixture was heated to 70°C and a preheated liposomal phyomelanins, obtained by dispersing at 20.000 rpm for 10 minutes the solutions of vegetal melanin with soybean lecithin and water, is added thereto.

The same procedure but with a 10% solution of caramel in NaOH 1N is carried out in Comparative Example 1.

The resultant mixtures are stirred and allowed to cool to room temperature.

### Test of suppression of UV-induced erythema by vegetal melanin

A comparative study on vegetal melanin radiation protectant activity on the suppression of UV-induced erythema is assessed according the method described by Bangha E; Elsner P; Kistler GS (Arch. Dermatol. Res., 288(9):522-6. 1996).

3 volunteers were topically treated by applying approximately 10 g of the composition of Example 6 in the right side of the back and 10 g of the composition without vegetal melanin (as per Comparative Example 1) on the controlateral side, then irradiated with 0.1 J/cm2 UVB on two 5-cm 2 areas on the lower back.

The UV-induced erythema was examined 24 h after irradiation by visual scoring. The differences in redness is shown in Table E.

**TABLE E Visual scoring by applying topical compositions with and without vegetal melanin**

| | Redness after 24 hours |
|---|---|
| Comparative Example 1 (right side, no vegetal melanin) | persistant |
| Example 14 (left side, with vegetal melanin) | none |

As it can be noted, the presence of vegetal melanin contributes to the prevention of UV-induced erythema.

### Applicative Example 7 - Oral composition with vegetal melanin

A composition is prepared in the form of ampoules with the following ingredients:

| | |
|---|---|
| Vegetal melanin solution of Example 11 | 2.5 g |
| ascorbic acid | 12.0 mg |
| maltol | 30.0 mg |
| L-tartaric acid | 15.0 mg |
| strawberry flavor | 2.0 mg |
| sodium citrate | 1.0 g |

solubilizing the mixture in distilled water qs to 250 ml.

It should be understood that the specific forms of the invention herein illustrated and described are intended to be representative only. Changes, including but not limited to those suggested in this specification, may be made in the illustrated embodiments without departing from the clear teachings of the disclosure.

Accordingly, reference should be made to the following appended claims in determining the full scope of the invention.

## Claims

1. Synthetic vegetal melanins obtainable by in vitro polymerization of at least one monomer unit (a), and optionally at least one compound (b), wherein:
(a) is a plant polyphenol; and
(b) is an eumelanin precursors;
wherein the plant polyphenol is selected among:
- a vegetal substance of formula (I): 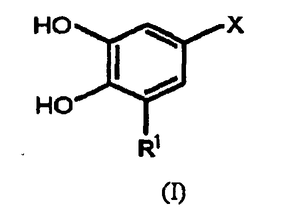
wherein:
R¹ represents hydrogen, hydroxy, or methoxy group; and
X represents a residue of formulas (II) or (III) 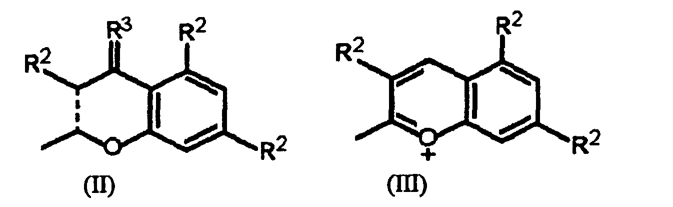
wherein:
R² represent, each independently, hydrogen, hydroxy, methoxy, or an O-glycosilated group;
R³ represents, one oxygen, two hydrogen atoms, or one hydrogen and one hydroxy group;
the phantom line between C₂-C₃ represents a double or a single bond,
- protocathechuic aldehyde;
- tannic acid;
- hydroxytyrosol, gallic acid and mixture thereof;
as well as salts, esters and solvates thereof,
and wherein the eumelanin precursor (b) is selected in the group consisting in L-dopa, DHI, DHICA, dopamine, pyrocatechol, pyrogallol and mixture thereof.

2. Synthetic vegetal melanins according to claim 1, wherein the plant polyphenol (a) of formula (I) with X being a residue of formula (II) is a flavonoid selected in the group consisting in quercetin, fisetin, fustin, luteolin, OPC, catechin, epicatechin, GC, GCG, EGC, EGCG, myricetin, dihydroquercetin and mixture thereof.

3. Synthetic vegetal melanins according to claim 1, wherein the plant polyphenol (a) of formula (I) with X being a residue of formula (III) is a flavonoid anthocyanins selected in the group consisting in cyanidin, delphinidin and mixture thereof.

4. Method of producing synthetic vegetal metanics according to claims 1 to 3 comprising bubbling air or oxygen through an alkaline aqueous solution of the monomers at pH 10 or higher, during 12 to 48 hours, at a temperature ranging from 10 to 90°C.

5. Method of claim 4, further comprising catalytic amounts of pro-oxidant metals selected in the group consisting in Cu⁺⁺, Fe⁺⁺, Ni⁺⁺, Co⁺⁺ and mixture thereof.

6. Method of claim 4 wherein a chemical oxidizing agent selected in the group consisting in hydrogen peroxide, hydrogen iodide, ammonium persulfate, potassium permanganate, magnesium perchlorate and mixture thereof, is used in lieu of air or oxygen.

7. Method of producing synthetic vegetal melanins according to claim 1 to 3 comprising bubbling air or oxygen through an buffered aqueous solution of the monomers at pH from 9.5 to 4.5, during 12 to 48 hours, at a temperature ranging from 20 to 45°C in presence of a suitable enzyme.

8. Method of claim 7, wherein the enzyme is selected in the group consisting in tyrosinases, polyphenoloxidases (catachol oxidases), phenolases, (phenoloxidases), peroxidases, laccases, lypoxygenase, and mixture thereof.

9. Method according to claims 7 to 8 wherein the monomer units (a) or (b) are formed in situ from the pre-momomers, bearing a monophenolic moiety

10. Method of claim 9 where the pre-momomers are selected in the group consisting in dihydrokaempferol, armadendrin, p-hydroxybenzaldehyde, PHBA, tyrosol, p-coumaric acid, apigenin, kaempferol, pelargonin, genistein, tyrosine, tyramine, 5-hydroxy-indole and mixture thereof.

11. Cosmetic composition comprising as active ingredient at least one synthetic vegetal melanins according to claims 1 to 3.

12. Cosmetic composition according to claim 11, for facial make-up, hair dyes, tanning, anti-sun, toiletry, moisturizing and protecting skin.

13. Pharmaceutical or nutritional composition having anti-inflammatory and immunomodulation activity which comprises as active ingredient at least a synthetic vegetal melanin according to claims 1 to 3.

## Patentansprüche

1. Synthetische Melanine auf pflanzlicher Basis, erhältlich durch *in vitro* Polymerisation mindestens einer Polymereinheit (a) und optional mindestens einer Verbindung (b), wobei:
(a) ein pflanzliches Polyphenol darstellt; und
(b) ein Eumelaninvorläufer ist;
wobei das pflanzliche Polyphenol ausgewählt ist aus:
- einer Substanz der Formel (I) auf pflanzlicher Basis: 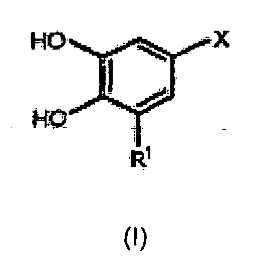
worin
R¹ Wasserstoff, Hydroxy oder eine Methoxygruppe darstellt; und
X einen Rest der Formeln (II) oder (III) darstellt 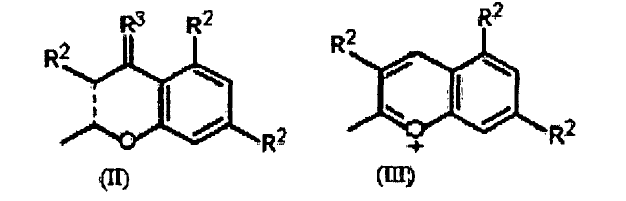
worin:
R² jeweils unabhängig voneinander Wasserstoff, Hydroxy, Methoxy oder eine O-glykosilierte Gruppe repräsentiert;
R³ einen Sauerstoff, zwei Wasserstoffatome oder ein Wasserstoff und eine Hydroxygruppe darstellt;
die Phantomlinie zwischen C₂ und C₃ für eine Doppelbindung oder eine Einfachbindung steht
- Protocatechualdehyd
- Gerbsäure
- Hydroxytyrosol, Gallussäure und deren Mischungen;
sowie deren Salzen, Estern und Solvaten,
und wobei der Eumelaninvorläufer (b) ausgewählt ist aus der Gruppe bestehend aus L-Dopa, DHI (5,6-Dihydroxyindol), DHICA (5,6-Dihydroxyindol-2-carbonsäure), Dopamin, Pyrocatechol, Pyrogallol und deren Mischungen.

2. Synthetische Melanine auf pflanzlicher Basis gemäß Anspruch 1, wobei es sich bei dem pflanzlichen Polyphenol (a) der Formel (I) mit einem Rest der Formel (II) als X um ein Flavonoid handelt, das ausgewählt ist aus der Gruppe bestehend aus Quercetin, Fisetin, Fustin, Luteolin, OPC (oligomerem Proanthocyanidin), Catechin, Epicatechin, GC ((+)-Gallocatechin), GCG ((+)-Gallocatechingallat), EGC ((-)-Epigallocatechin), EGCG ((-)-Epigallocatechingallat), Myricetin, Dihydroquercetin und einer Mischung daraus.

3. Synthetische Melanine auf pflanzlicher Basis gemäß Anspruch 1, wobei es sich bei dem Polyphenol auf pflanzlicher Basis (a) der Formel (I) mit einem Rest der Formel (III) als X um ein Flavonoidanthocyanin ausgewählt aus der Gruppe bestehend aus Cyanidin, Delphinidin und deren Mischungen handelt.

4. Verfahren zur Herstellung synthetischer Melanine auf pflanzlicher Basis gemäß den Ansprüchen 1 bis 4 umfassend das Durchperlenlassen von Luft oder Sauerstoff durch eine alkalische wässrige Lösung des Monomers bei einem pH von 10 oder höher für einen Zeitraum von 12 bis 48 Stunden bei einer von 10 bis 90°C reichenden Temperatur.

5. Verfahren nach Anspruch 4, des weiteren umfassend katalytische Mengen eines Metalls als Prooxidationsmittel ausgewählt aus der Gruppe betsehend aus Cu⁺⁺, Fe⁺⁺, Ni⁺⁺, Co⁺⁺ und deren Mischungen.

6. Verfahren nach Anspruch 4, wobei ein chemisches Oxidationsmittel ausgewählt aus der Gruppe bestehend aus Wasserstoffperoxid, lodwasserstoff, Ammoniumpersulfat, Kaliumpermanganat, Magnesiumperchlorat und deren Mischungen anstelle von Luft oder Sauerstoff verwendet wird.

7. Verfahren zur Herstellung von synthetischen Melaninen auf pflanzlicher Basis gemäß den Ansprüchen 1 bis 3, umfassend das Durchperlenlassen von Luft oder Sauerstoff durch eine gepufferte wässrige Lösung des Monomers bei einem pH von 9,5 bis 4,5 für einen Zeitraum von 12 bis 48 Stunden bei einer von 20 bis 45°C reichenden Temperatur in der Gegenwart eines geeigneten Enzyms.

8. Verfahren nach Anspruch 7 wobei das Enzym ausgewählt ist aus der Gruppe bestehend aus Tyrosinasen, Polyphenoloxidasen (Catecholoxidasen), Phenolasen (Phenoloxidasen), Peroxidasen, Laccasen, Lypoxygenasen und deren Mischungen.

9. Verfahren nach den Ansprüchen 7 bis 8 wobei die Monomereinheiten (a) oder (b) *in situ* aus den Vormonomeren, die einen monophenolischen Anteil tragen, gebildet werden.

10. Verfahren nach Anspruch 9 wobei die Vormonomere ausgewählt sind aus der Gruppe bestehend aus Dihydrokämpferol, Armadendrin, p-Hydroxybenzaldehyd, PHB (PHBA, Parahydroxybenzoesäure), Tyrosol, p-Cumarsäure, Apigenin, Kämpferol, Pelargonin, Genistein, Tyrosin, Tyramin, 5-Hydroxyindol und deren Mischungen.

11. Kosmetische Zusammensetzung umfassend mindestens ein synthetisches Melanin auf pflanzlicher Basis gemäß den Ansprüchen 1 bis 3 als aktiven Bestandteil.

12. Kosmetische Zusammensetzung gemäß Anspruch 11 für Gesichts-Make-up, für Haartönungen, zum Bräunen, zum Spenden von Feuchtigkeit für die Haut und zum Schützen der Haut, als Sonnenschutz und als Toilettenartikel.

13. Pharmazeutische Zusammensetzung oder Nahrungszusammensetzung mit anti-inflammatorischer und immunomodulierender Aktivität, die mindestens ein synthetisches Melanin auf pflanzlicher Basis gemäß den Ansprüchen 1 bis 3 enthält.

## Revendications

1. Mélanines végétales synthétiques pouvant être obtenues par polymérisation *in vitro* d'au moins un motif monomère (a), et éventuellement d'au moins un composé (b), où :
(a) est un polyphénol végétal ; et
(b) est un précurseur d'eumélanine ;
où le polyphénol végétal est choisi parmi :
- une substance végétale de formule (I) : 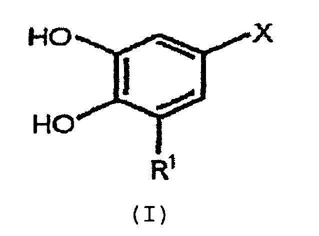
dans laquelle :
R¹ représente l'hydrogène ou un groupe hydroxy ou méthoxy ; et
X représente un résidu de formule (II) ou (III) 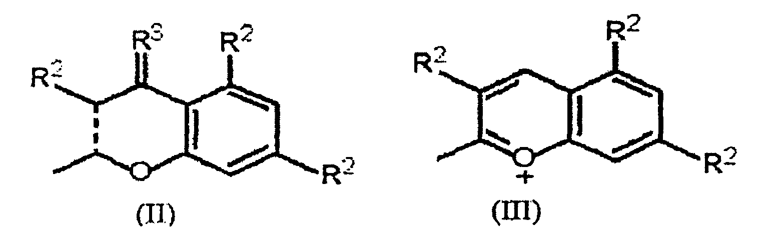
où :
chaque R² représente indépendamment l'hydrogène ou un groupe hydroxy, méthoxy ou O-glycosylé;
R³ représente un oxygène, deux atomes d'hydrogène, ou un hydrogène et un groupe hydroxy;
la ligne de tirets entre C₂-C₃ représente une liaison double ou simple,
- l'aldéhyde protocatéchuique ;
- l'acide tannique ;
- l'hydroxytyrosol, l'acide gallique et leur mélange ;
ainsi que leurs sels, esters et solvates,
et où le précurseur d'eumélanine (b) est choisi dans le groupe constitué par la L-dopa, le DHI, le DHICA, la dopamine, le pyrocatéchol, le pyrrogallol et leur mélange.

2. Mélanines végétales synthétiques selon la revendication 1, dans lesquelles le polyphénol végétal (a) de formule (I) où X est un résidu de formule (II) est un flavonoïde choisi dans le groupe constitué par la quercétine, la fisétine, la fustine, la lutéoline, l'OPC, la catéchine, l'épicatéchine, le GC, le GCG, l'EGC, l'EGCG, la myricétine, la dihydroquercétine et leur mélange.

3. Mélanines végétales synthétiques selon la revendication 1, dans lesquelles le polyphénol végétal (a) de formule (I) où X est un résidu de formule (III) est une anthocyanine flavonoïde choisie dans le groupe constitué par la cyanidine, la delphinidine et leur mélange.

4. Procédé pour produire des mélanines végétales synthétiques selon les revendications 1 à 4, consistant à faire barboter de l'air ou de l'oxygène dans une solution aqueuse alcaline des monomères à un pH de 10 ou plus, durant 12 à 48 heures, à une température située dans la plage allant de 10 à 90°C.

5. Procédé selon la revendication 4, comprenant en outre des quantités catalytiques de métaux pro-oxydants choisis dans le groupe constitué par Cu⁺⁺, Fe⁺⁺, Ni⁺⁺, Co⁺⁺ et leur mélange.

6. Procédé selon la revendication 4, dans lequel un agent oxydant chimique choisi dans le groupe constitué par le peroxyde d'hydrogène, l'iodure d'hydrogène, le persulfate d'ammonium, le permanganate de potassium, le perchlorate de magnésium et leur mélange, est utilisé au lieu de l'air ou de l'oxygène.

7. Procédé pour produire des mélanines végétales synthétiques selon les revendications 1 à 3, consistant à faire barboter de l'air ou de l'oxygène dans une solution aqueuse tamponnée des monomères à un pH de 9,5 à 4,5, durant 12 à 48 heures, à une température située dans la plage allant de 20 à 45°C en présence d'une enzyme convenable.

8. Procédé selon la revendication 7, dans lequel l'enzyme est choisie dans le groupe constitué par les tyrosinases, les polyphénoloxydases (catachol oxydases), les phénolases (phénol oxydases), les peroxydases, les laccases, la lipoxygénase, et leur mélange.

9. Procédé selon les revendications 7 et 8, dans lequel les motifs monomères (a) ou (b) sont formés *in situ* à partir des pré-monomères, portant un fragment monophénolique.

10. Procédé selon la revendication 9, dans lequel les pré-monomères sont choisis dans le groupe constitué par le dihydrokaempférol, l'armadendrine, le p-hydroxybenzaldéhyde, le PHBA, le tyrosol, l'acide p-coumarique, l'apigénine, la kaempférol, la pélargonine, la génistéine, la tyrosine, la tyramine, le 5-hydroxyindole et leur mélange.

11. Composition cosmétique comprenant, à titre d'ingrédient actif, au moins une des mélanines végétales synthétiques selon les revendications 1 à 3.

12. Composition cosmétique selon la revendication 11, pour le maquillage du visage, la teinture des cheveux, le bronzage, la protection solaire, la toilette, l'hydratation et la protection de la peau.

13. Composition pharmaceutique ou nutritionnelle ayant une activité anti-inflammatoire et immuno-modulatrice, qui comprend, à titre d'ingrédient actif, au moins une mélanine végétale synthétique selon les revendications 1 à 3.
